(19) Europäisches Patentamt
European Patent Office
Office européen des brevets

(11) Veröffentlichungsnummer : **0 379 911 B1**

(12) # EUROPÄISCHE PATENTSCHRIFT

(45) Veröffentlichungstag der Patentschrift : **02.12.92 Patentblatt 92/49**

(51) Int. Cl.⁵ : **C07D 231/56,** A01N 43/56

(21) Anmeldenummer : **90100662.7**

(22) Anmeldetag : **13.01.90**

(54) **N-Phenyltetrahydroindazolderivate.**

Die Akte enthält technische Angaben, die nach dem Eingang der Anmeldung eingereicht wurden und die nicht in dieser Patentschrift enthalten sind.

(30) Priorität : **21.01.89 DE 3901705**

(43) Veröffentlichungstag der Anmeldung : **01.08.90 Patentblatt 90/31**

(45) Bekanntmachung des Hinweises auf die Patenterteilung : **02.12.92 Patentblatt 92/49**

(84) Benannte Vertragsstaaten : **BE CH DE ES FR GB IT LI NL**

(56) Entgegenhaltungen :
**EP-A- 0 105 721**
**PATENT ABSTRACTS OF JAPAN, Band 9, Nr. 21 (C-263)[1744], 29. Januar 1985; & JP-A-59 170 071**
**PATENT ABSTRACTS OF JAPAN, Band 10, Nr. 194 (C-358)[2250], 8. Juli 1986; & JP-A-61 36 268**

(73) Patentinhaber : **BASF Aktiengesellschaft**
**Carl-Bosch-Strasse 38**
**W-6700 Ludwigshafen (DE)**

(72) Erfinder : **Rueb, Lothar, Dr.**
**Am Schoeneck 11**
**W-6720 Speyer (DE)**
Erfinder : **Eicken, Karl, Dr.**
**Am Huettenwingert 12**
**W-6706 Wachenheim (DE)**
Erfinder : **Plath, Peter, Dr.**
**Hans-Balcke-Strasse 13**
**W-6710 Frankenthal (DE)**
Erfinder : **Westphalen, Karl-Otto, Dr.**
**Mausbergweg 58**
**W-6720 Speyer (DE)**
Erfinder : **Wuerzer, Bruno, Dr.**
**Ruedigerstrasse 13**
**W-6701 Otterstadt (DE)**

EP 0 379 911 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft N-Phenyltetrahydroindazolderivate der allgemeinen Formel I

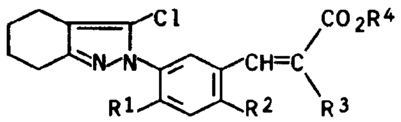

I

in der die Substituenten folgende Bedeutung haben:

$R^1$ Wasserstoff oder Fluor,

$R^2$ ein Halogenatom,

$R^3$ Wasserstoff, ein Halogenatom oder eine $C_1$-$C_4$-Alkylgruppe,

$R^4$ Wasserstoff, eine $C_1$-$C_6$-Alkylgruppe, welche durch ein oder zwei $C_1$-$C_4$-Alkoxygruppen substituiert sein kann, eine $C_3$-$C_6$-Alkenylgruppe, eine $C_3$-$C_6$-Alkinylgruppe oder ein Benzylrest,

wobei die Formel I sämtliche isomeren Formen dieser Verbindungen umfaßt.

Des weiteren betrifft die Erfindung Verfahren zur Herstellung dieser Verbindungen sowie deren Verwendung als herbizide Mittel.

Aus der EP-A 105 721 sind u.a. herbizide N-Phenyltetrahydroindazolderivate I' bekannt

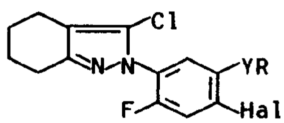

I'

in der die Reste beispielsweise folgende Bedeutung haben:

Hal Chlor oder Brom

Y Sauerstoff oder eine Iminogruppe

R eine $C_1$-$C_4$-Alkylgruppe, eine $C_3$-$C_4$-Alkenylgruppe, eine $C_3$-$C_4$-Alkinylgruppe, eine $C_1$-$C_6$-Alkoxycarbonylmethylgruppe, eine $C_3$-$C_6$-Cycloalkoxycarbonylmethylgruppe oder eine $C_1$-$C_4$-Halogenalkoxycarbonylmethylgruppe.

Ferner werden in den Patent Abstracts of Japan 10 (1986), Nr. 194 (C-358) [2250] herbizid wirksame Tetrahydroindazole der Formel I''

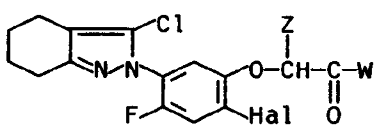

I''

beschrieben, wobei die Substituenten definitionsgemäß folgende Bedeutungen haben:

Hal Chlor oder Brom,

Z Wasserstoff oder Alkyl und

W eine Alkoxy- oder Alkenyloxygruppe, die Aminogruppe, eine Alkyl- oder Alkenylaminogruppe, eine Dialkyl- oder Dialkenylaminogruppe, eine N-Alkyl-N-alkenyl-aminogruppe oder eine azaheterocyclische Gruppe, die noch ein Sauerstoffatom als Ringglied enthalten kann.

Schließlich sind den Patent Abstracts of Japan 9 (1985), Nr. 21 (C-263) [1744] 2-Phenyl-4,5,6,7-tetrahydro-2H-indazol-Derivate der Formel I'''

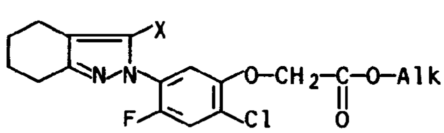

I'''

(X = Chlor oder Methyl und Alk = Alkyl oder Halogenalkyl) zu entnehmen. Für diese Verbindungen wird ebenfalls

eine herbizide Wirkung angegeben.

Besonders für die Anwendung in Pflanzenkulturen sind jedoch Verbindungen wünschenswert, die bei geringerer Aufwandmenge unerwünschte Pflanzen besser bekämpfen ohne die Kulturpflanzen nennenswert zu schädigen (Selektivität).

Der Erfindung lagen daher neue N-Phenyltetrahydroindazolderivate mit verbesserter herbizider Wirksamkeit als Aufgabe zugrunde.

Demgemäß wurden die eingangs definierten N-Phenyltetrahydroindazolderivate I gefunden.

Außerdem wurden Verfahren zur Herstellung der Verbindungen I, die Verwendung dieser Verbindungen als Herbizide sowie diese enthaltende herbizide Mittel gefunden.

Man erhält die Verbindungen I beispielsweise dadurch, daß man ein Indazolzimtsäurederivat VII in einem aprotisch polaren organischen Lösungsmittel in an sich bekannter Weise (Houben Weyl Bd. VIII, 545, 655; X/2, 747) in Anwesenheit einer Base mit einer Verbindung VIII umsetzt.

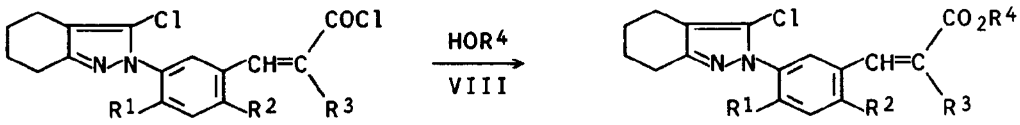


VII I

Als Lösungsmittel für diese Umsetzung dienen vornehmlich höhersiedende Kohlenwasserstoffe wie Xylol und Toluol, Carbonsäureester wie Essigsäureethylester und Ether wie Dioxan und Tetrahydrofuran.

Die Umsetzung erfolgt in der Regel bei Temperaturen von (-10)°C bis 200°C, vorzugsweise bei 0 bis 150°C.

Geeignete Basen für diese Umsetzung sind beispielsweise tertiäre Amine wie Triethylamin und Pyridin oder anorganische Salze wie Natriumhydroxid, Kaliumhydroxid und Kaliumcarbonat.

Die für dieses Verfahren benötigten Indazolzimtsäurederivate VII sind in fünf unabhängigen Reaktionsstufen gemäß dem folgenden Reaktionsschema zugänglich.

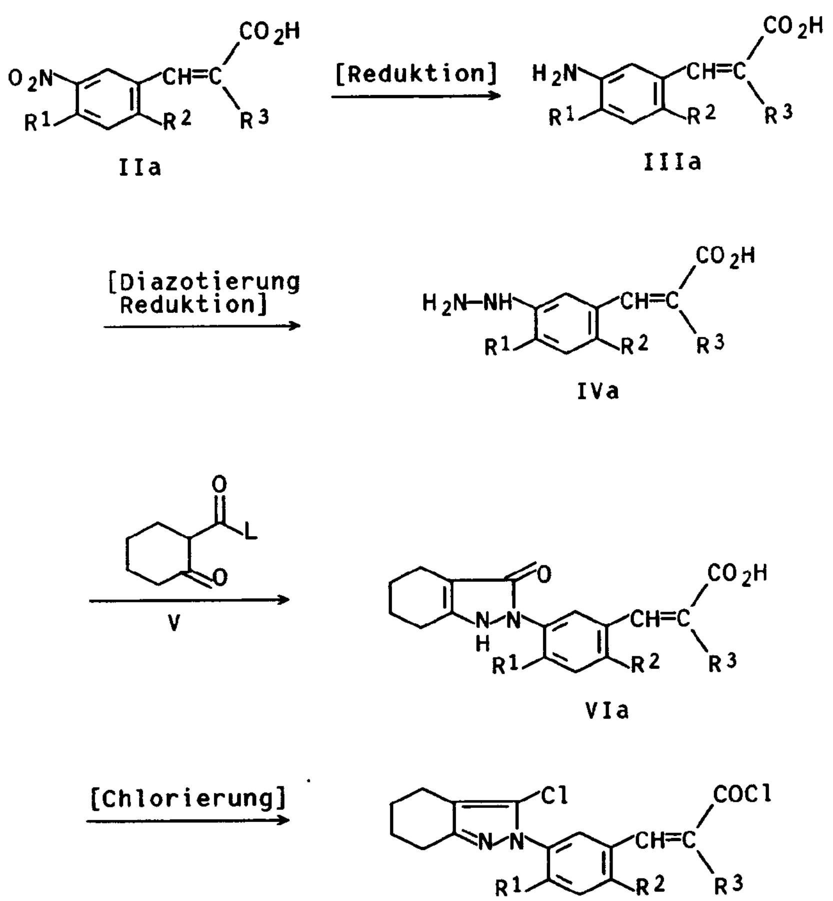


IIa IIIa

IVa

VIa

VII

In der ersten Stufe der Reaktionsfolge wird eine Nitrozimtsäure IIa in an sich bekannter Weise mit anorganischen Verbindungen wie Zinn-II-salzen oder Eisen oder, sofern $R^3$ nicht Halogen bedeutet, durch katalytische Hydrierung an Metallkontakten wie Raney-Nickel, Palladium und Platin in einem inerten organischen Lösungsmittel zum Anilinderivat IIIa reduziert.

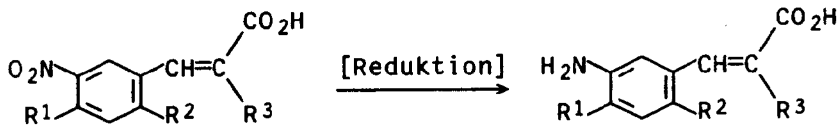


IIa IIIa

Geeignete Lösungsmittel für die Reduktion mit anorganischen Verbindungen sind beispielsweise Alkohole wie Methanol, Ethanol und Isopropanol und niedere Alkansäuren wie Ameisensäure, Essigsäure und Propionsäure sowie entsprechende Gemische. Die Reaktionstemperaturen liegen bei 25°C bis 150°C, vorzugsweise bei 25°C bis 100°C.

Sofern man die Reduktion an Metallkontakten mit Wasserstoff durchführt, eignen sich Lösungsmittel wie Methanol, Tetrahydrofuran und Eisessig und entsprechende Gemische bei einem Wasserstoffdruck von 1 bis 150 bar, vorzugsweise 1 bis 50 bar und Temperaturen von 25°C bis 100°C, vorzugsweise 25 bis 70°C.

Das so erhaltene Anilinderivat IIIa wird dann in an sich bekannter Weise in einem inerten Lösungsmittel mit einem anorganischen oder organischen Nitrit zum Aryldiazonium-Salz diazotiert, welches in situ mit einem anorganischen Reduktionsmittel zum Hydrazinozimtsäurederivat IVa reduziert wird.

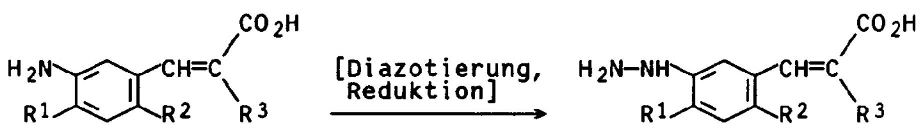


IIIa IVa

Die Wahl des Lösungsmittels richtet sich hierbei nach der Art des Nitrits.

So werden anorganische Nitrite wie salpetrige Säure und deren Alkalimetall- und Erdalkalimetallsalze vorzugsweise in wäßriger Lösung in Anwesenheit von Mineralsäuren bei Temperaturen von (-30)°C bis 50°C, vorzugsweise (-10)°C bis 5°C, eingesetzt.

Bedient man sich organischer Nitrite wie Amylnitrit, so werden aprotische Lösungsmittel wie Toluol bevorzugt. Die Reaktionstemperatur liegt in diesem Fall bei (-10)°C bis 25°C.

Die anschließende Reduktion erfolgt in jedem Fall in situ mit anorganischen Reduktionsmitteln wie Zinn-II-salzen, Natriumdithionit, Alkalimetallsulfiten und -hydrogensulfiten wie Natriumsulfit und Natriumhydrogensulfit und/oder mit Schwefeldioxid. Zur Lösungsvermittlung kann es von Vorteil sein, ein Lösungsmittel wie Eisessig, Ethanol oder Toluol zuzusetzen.

In der nächsten Reaktionsstufe wird das Hydrazinzimtsäurederivat IVa in an sich bekannter Weise in einem inerten organischen Lösungsmittel bei Temperaturen bis 200°C, vorzugsweise 25°C bis 150°C, mit einem Cyclohexanoncarbonsäurederivat V zum Indazolzimtsäurederivat VIa cyclisiert.

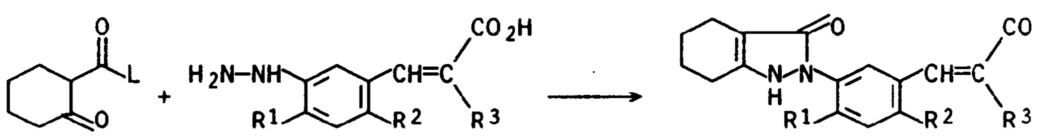

V IVa VIa

In der Formel V bedeutet L eine nucleophile Abgangsgruppe wie Halogen z.B. Chlor und Brom oder Alkoholat wie Methylat, Ethylat, Propylat, iso-Propylat und Tosylat.

Vorzugsweise arbeitet man hier in Lösungsmitteln wie niederen Alkansäuren wie Essigsäure oder in aprotischen Lösungsmitteln wie Toluol und Xylol. Sofern L in der Formel V Halogen wie Chlor und Brom bedeutet, kann es von Vorteil sein, in Anwesenheit eines tertiären Amins als Base zu arbeiten. Geeignete Basen sind dafür beispielsweise Triethylamin, Diisopropylethylamin, N,N-Dimethylanilin, N,N-Dimethyl-p-aminopyridin, Pyridin, Isochinolin, N-Methylpyrolidin, N,N,N′,N′-Tetramethylethylendiamin, 1,5-Diazabicyclo (4,3,0)non-5-en

und 1,8-Diazabicyclo(5,4,0)undec-7-en.

Aus diesem Indazolzimtsäurederivat VIa erhält man in an sich bekannter Weise durch Umsetzung mit einem in der organischen Chemie üblichen Chlorierungsmittel in Anwesenheit oder Abwesenheit eines inerten organischen Lösungsmittels sowie in Anwesenheit oder Abwesenheit einer Base das Indazolzimtsäurechlorid VII.

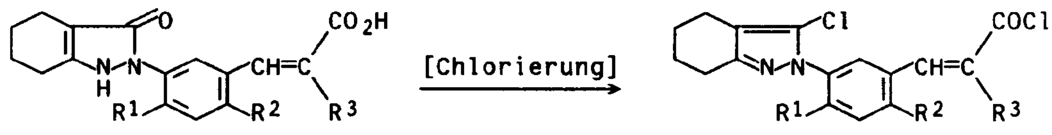


VIa VII

Geeignete Chlorierungsmittel sind z.B. Oxychloride wie Phosphoroxytrichlorid, Thionylchlorid oder Phosgen, Trichlormethylchloroformiat oder Chloride wie Phosphortrichlorid, Phosphorpentachlorid oder Schwefeltetrachlorid. Vorzugsweise verwendet man Phosphoroxytrichlorid.

Die Reaktion kann bei Temperaturen von 25 bis 200°C, vorzugsweise 60 bis 160°C, gegebenenfalls in Anwesenheit einer Base durchgeführt werden.

Für diese Umsetzung geeignete Basen sind z.B. tertiäre Amine wie die oben genannten.

Als Lösungsmittel kommen hier beispielsweise Toluol, Xylol und Chloroform zur Anwendung.

Man erhält die Verbindungen I aber auch, indem man ein entsprechendes Nitrozimtsäurederivat 11 in an sich bekannter Weise analog den für die Nitrozimtsäure IIa geschilderten Reaktionsstufen gemäß dem folgenden Schema umsetzt.

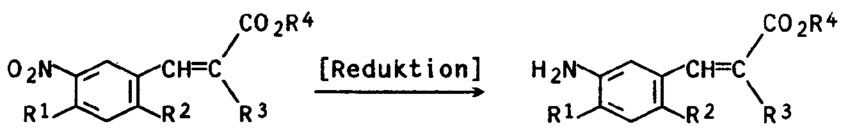


II III

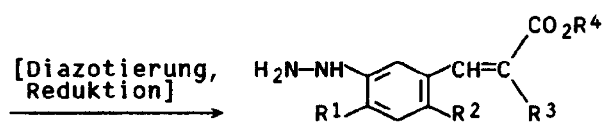


IV

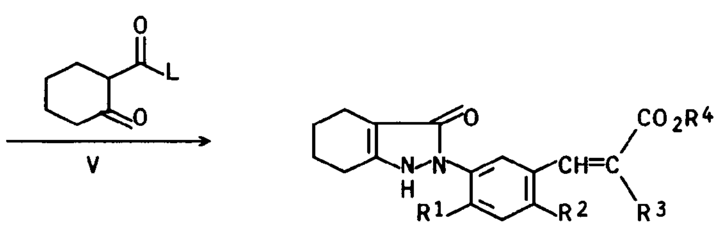


VI

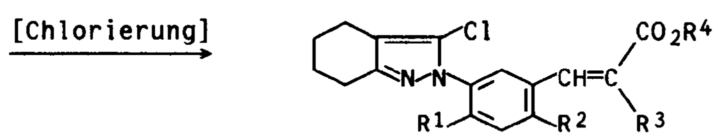


I

Die einzelnen Stufen dieser Synthese werden im allgemeinen entsprechend den vorstehend für das Zimtsäurederivat geschilderten Bedingungen durchgeführt.

Insbesondere führt man die Reduktion des Nitrozimtsäurederivates II in einem protisch polaren Lösungsmittel in Gegenwart eines anorganischen Reduktionsmittels bei einer Temperatur von 25°C bis 150°C, vorzugsweise 40°C bis 100°C, durch.

Als Lösungsmittel kommen hierbei Carbonsäuren wie Essigsäure, Propionsäure und iso-Buttersäure sowie Alkohole wie Methanol, Ethanol und iso-Propanol sowie entsprechende Gemische in Betracht.

Als Reduktionsmittel dienen vorzugsweise Zinn-II-salze oder Eisen.

Die Diazotierung und Reduktion der Aminozimtsäurederivate III zu den entsprechenden Hydrazinozimtsäurederivaten IV wird vorzugsweise in wäßriger Lösung in Anwesenheit von Mineralsäuren wie Salzsäure oder Carbonsäuren wie Essigsäure mit Diazotierungsmitteln wie salpetriger Säure oder Natrium- bzw. Kaliumnitrit und Reduktionsmitteln wie Zinn-II-salzen oder den vorstehend genannten Sulfiten bei einer Temperatur von (-15)°C bis + 5°C durchgeführt.

Die anschließende Cyclisierung der Hydrazinozimtsäurederivate IV zu den entsprechenden Indazolzimtsäurederivaten VI führt man vorzugsweise mit Cyclohexanoncarbonsäurederivaten V durch, in denen L eine Alkoxygruppe wie die Methoxy-, Ethoxy- und die iso-Propyloxygruppe bedeutet.

Als Lösungsmittel eignen sich hierbei besonders niedere Carbonsäuren wie Essigsäure und Propionsäure. Die bevorzugten Reaktionstemperaturen liegen bei 50°C bis 100°C.

Als Chlorierungsmittel für die Umsetzung der Indazolzimtsäurederivate VI zu den erfindungsgemäßen N-Phenyltetrahydroindazolderivaten I eignen sich insbesondere Phosgen, Phosphoroxytrichlorid und Trichlormethylchloroformiat. Die Reaktion kann vorzugsweise ohne Lösungsmittel oder in Toluol, Xylol, Dimethylformamid oder Chloroform als Lösungsmittel bei Temperaturen von 60°C bis 160°C durchgeführt werden.

Im Hinblick auf die bestimmungsgemäße Verwendung der Verbindungen I als Herbizide kommen als Substituenten vorzugsweise folgende Reste in Betracht:

$R^1$ Wasserstoff oder Fluor,

$R^2$ Halogen wie Fluor, Chlor oder Brom, insbesondere Chlor,

$R^3$ Wasserstoff;

ein Halogenatom wie unter $R^2$ genannt, insbesondere Chlor und Brom;

eine Alkylgruppe wie Methyl, Ethyl, Propyl, 1-Methylethyl, Butyl, 1-Methylpropyl, 2-Methylpropyl und 1,1-Dimethylethyl, insbesondere Methyl und Ethyl;

$R^4$ Wasserstoff,

eine $C_1$-$C_6$-Alkylgruppe wie die unter $R^3$ genannten sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, insbesondere Methyl, Ethyl, Propyl und iso-Propyl, wobei diese Alkylgruppe durch ein bis zwei $C_1$-$C_4$-Alkoxygruppen wie Methoxy, Ethoxy, Propoxy, 1-Methylethoxy, Butoxy, 1-Methylpropoxy, 2-Methylpropoxy und 1,1-Dimethylethoxy, substituiert sein kann, wobei Alkoxyalkylgruppen wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl, besonders bevorzugt sind;

eine Alkenylgruppe wie 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,2-Dimethyl-1-butenyl, 1,3-Dimethyl-2-butenyl, 1,2-Dimethyl-2-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3 -butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl,

eine Alkinylgruppe wie 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl,

1,1-Dimethyl-3-butinyl, 1,2-Dimethyl-3-butinyl, 2,2-Dimethyl -3-butinyl, 3,3-Dimethyl-1-butinyl, 1-Ethyl-2-butinyl, 1-Ethyl-3 -butinyl, 2-Ethyl-3-butinyl und 1-Ethyl-1-methyl-2-propinyl,

sowie ein Benzylrest

Beispiele für sehr aktive Verbindungen I sind in der nachstehenden Tabelle 1 aufgeführt.

Tabelle 1

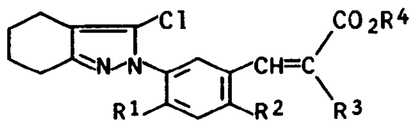

I

| $R^1$ | $R^2$ | $R^3$ | $R^4$ |
|---|---|---|---|
| H | Cl | H | H |
| F | Cl | H | H |
| H | Br | H | H |
| F | Br | H | H |
| H | Cl | Cl | H |
| F | Cl | Cl | H |
| H | Br | Cl | H |
| F | Br | Cl | H |
| H | Cl | Br | H |
| F | Cl | Br | H |
| H | Br | Br | H |
| F | Br | Br | H |
| H | Cl | $CH_3$ | H |
| F | Cl | $CH_3$ | H |
| H | Br | $CH_3$ | H |
| F | Br | $CH_3$ | H |
| H | Cl | $CH_2CH_3$ | H |
| F | Cl | $CH_2CH_3$ | H |
| H | Br | $CH_2CH_3$ | H |
| F | Br | $CH_2CH_3$ | H |
| H | Cl | H | $CH_3$ |
| F | Cl | H | $CH_3$ |
| H | Br | H | $CH_3$ |
| F | Br | H | $CH_3$ |
| H | Cl | Cl | $CH_3$ |
| F | Cl | Cl | $CH_3$ |
| H | Br | Cl | $CH_3$ |
| F | Br | Cl | $CH_3$ |
| H | Cl | Br | $CH_3$ |
| F | Cl | Br | $CH_3$ |
| H | Br | Br | $CH_3$ |
| F | Br | Br | $CH_3$ |
| H | Cl | $CH_3$ | $CH_3$ |
| F | Cl | $CH_3$ | $CH_3$ |
| H | Br | $CH_3$ | $CH_3$ |
| F | Br | $CH_3$ | $CH_3$ |
| H | Cl | $CH_2CH_3$ | $CH_3$ |

Tabelle 1 (Fortsetzung)

| $R^1$ | $R^2$ | $R^3$ | $R^4$ |
|---|---|---|---|
| F | Cl | $CH_2CH_3$ | $CH_3$ |
| H | Br | $CH_2CH_3$ | $CH_3$ |
| F | Br | $CH_2CH_3$ | $CH_3$ |
| H | Cl | H | $CH_2CH_3$ |
| F | Cl | H | $CH_2CH_3$ |
| H | Br | H | $CH_2CH_3$ |
| F | Br | H | $CH_2CH_3$ |
| H | Cl | Cl | $CH_2CH_3$ |
| F | Cl | Cl | $CH_2CH_3$ |
| H | Br | Cl | $CH_2CH_3$ |
| F | Br | Cl | $CH_2CH_3$ |
| H | Cl | Br | $CH_2CH_3$ |
| F | Cl | Br | $CH_2CH_3$ |
| H | Br | Br | $CH_2CH_3$ |
| F | Br | Br | $CH_2CH_3$ |
| H | Cl | $CH_3$ | $CH_2CH_3$ |
| F | Cl | $CH_3$ | $CH_2CH_3$ |
| H | Br | $CH_3$ | $CH_2CH_3$ |
| F | Br | $CH_3$ | $CH_2CH_3$ |
| H | Cl | $CH_2CH_3$ | $CH_2CH_3$ |
| F | Cl | $CH_2CH_3$ | $CH_2CH_3$ |
| H | Br | $CH_2CH_3$ | $CH_2CH_3$ |
| F | Br | $CH_2CH_3$ | $CH_2CH_3$ |
| H | Cl | H | $(CH_2)_2CH_3$ |
| F | Cl | H | $(CH_2)_2CH_3$ |
| H | Cl | Cl | $(CH_2)_2CH_3$ |
| F | Cl | Cl | $(CH_2)_2CH_3$ |
| H | Cl | Br | $(CH_2)_2CH_3$ |
| F | Cl | Br | $(CH_2)_2CH_3$ |
| H | Cl | $CH_3$ | $(CH_2)_2CH_3$ |
| F | Cl | $CH_3$ | $(CH_2)_2CH_3$ |
| H | Cl | $CH_2CH_3$ | $(CH_2)_2CH_3$ |
| F | Cl | $CH_2CH_3$ | $(CH_2)_2CH_3$ |
| H | Cl | H | $CH(CH_3)_2$ |
| F | Cl | H | $CH(CH_3)_2$ |
| H | Cl | Cl | $CH(CH_3)_2$ |
| F | Cl | Cl | $CH(CH_3)_2$ |
| H | Cl | Br | $CH(CH_3)_2$ |
| F | Cl | Br | $CH(CH_3)_2$ |

Tabelle 1 (Fortsetzung)

| $R^1$ | $R^2$ | $R^3$ | $R^4$ |
|---|---|---|---|
| H | Cl | $CH_3$ | $CH(CH_3)_2$ |
| F | Cl | $CH_3$ | $CH(CH_3)_2$ |
| H | Cl | $CH_2CH_3$ | $CH(CH_3)_2$ |
| F | Cl | $CH_2CH_3$ | $CH(CH_3)_2$ |
| H | Cl | H | $(CH_2)_3CH_3$ |
| F | Cl | H | $(CH_2)_3CH_3$ |
| H | Cl | Cl | $(CH_2)_3CH_3$ |
| F | Cl | Cl | $(CH_2)_3CH_3$ |
| H | Cl | Br | $(CH_2)_3CH_3$ |
| F | Cl | Br | $(CH_2)_3CH_3$ |
| H | Cl | $CH_3$ | $(CH_2)_3CH_3$ |
| F | Cl | $CH_3$ | $(CH_2)_3CH_3$ |
| H | Cl | $CH_2CH_3$ | $(CH_2)_3CH_3$ |
| F | Cl | $CH_2CH_3$ | $(CH_2)_3CH_3$ |
| H | Cl | H | $CH_2CH(CH_3)_2$ |
| F | Cl | H | $CH_2CH(CH_3)_2$ |
| H | Cl | Cl | $CH_2CH(CH_3)_2$ |
| F | Cl | Cl | $CH_2CH(CH_3)_2$ |
| H | Cl | Br | $CH_2CH(CH_3)_2$ |
| F | Cl | Br | $CH_2CH(CH_3)_2$ |
| H | Cl | $CH_3$ | $CH_2CH(CH_3)_2$ |
| F | Cl | $CH_3$ | $CH_2CH(CH_3)_2$ |
| H | Cl | $CH_2CH_3$ | $CH_2CH(CH_3)_2$ |
| F | Cl | $CH_2CH_3$ | $CH_2CH(CH_3)_2$ |
| H | Cl | H | $(CH_2)_4CH_3$ |
| F | Cl | H | $(CH_2)_4CH_3$ |
| H | Cl | Cl | $(CH_2)_4CH_3$ |
| F | Cl | Cl | $(CH_2)_4CH_3$ |
| H | Cl | Br | $(CH_2)_4CH_3$ |
| F | Cl | Br | $(CH_2)_4CH_3$ |
| H | Cl | $CH_3$ | $(CH_2)_4CH_3$ |
| F | Cl | $CH_3$ | $(CH_2)_4CH_3$ |
| H | Cl | $CH_2CH_3$ | $(CH_2)_4CH_3$ |
| F | Cl | $CH_2CH_3$ | $(CH_2)_4CH_3$ |
| H | Cl | H | $(CH_2)_2CH(CH_3)_2$ |
| F | Cl | H | $(CH_2)_2CH(CH_3)_2$ |
| H | Cl | Cl | $(CH_2)_2CH(CH_3)_2$ |
| F | Cl | Cl | $(CH_2)_2CH(CH_3)_2$ |
| H | Cl | Br | $(CH_2)_2CH(CH_3)_2$ |

Tabelle 1 (Fortsetzung)

| $R^1$ | $R^2$ | $R^3$ | $R^4$ |
|---|---|---|---|
| F | Cl | Br | $(CH_2)_2CH(CH_3)_2$ |
| H | Cl | $CH_3$ | $(CH_2)_2CH(CH_3)_2$ |
| F | Cl | $CH_3$ | $(CH_2)_2CH(CH_3)_2$ |
| H | Cl | $CH_2CH_3$ | $(CH_2)_2CH(CH_3)_2$ |
| F | Cl | $CH_2CH_3$ | $(CH_2)_2CH(CH_3)_2$ |
| H | Cl | H | $(CH_2)_2OCH_3$ |
| F | Cl | H | $(CH_2)_2OCH_3$ |
| H | Cl | Cl | $(CH_2)_2OCH_3$ |
| F | Cl | Cl | $(CH_2)_2OCH_3$ |
| H | Cl | Br | $(CH_2)_2OCH_3$ |
| F | Cl | Br | $(CH_2)_2OCH_3$ |
| H | Cl | $CH_3$ | $(CH_2)_2OCH_3$ |
| F | Cl | $CH_3$ | $(CH_2)_2OCH_3$ |
| H | Cl | $CH_2CH_3$ | $(CH_2)_2OCH_3$ |
| F | Cl | $CH_2CH_3$ | $(CH_2)_2OCH_3$ |
| H | Cl | H | $CH(CH_3)CH_2OCH_3$ |
| F | Cl | H | $CH(CH_3)CH_2OCH_3$ |
| H | Cl | Cl | $CH(CH_3)CH_2OCH_3$ |
| F | Cl | Cl | $CH(CH_3)CH_2OCH_3$ |
| H | Cl | Br | $CH(CH_3)CH_2OCH_3$ |
| F | Cl | Br | $CH(CH_3)CH_2OCH_3$ |
| H | Cl | $CH_3$ | $CH(CH_3)CH_2OCH_3$ |
| F | Cl | $CH_3$ | $CH(CH_3)CH_2OCH_3$ |
| H | Cl | $CH_2CH_3$ | $CH(CH_3)CH_2OCH_3$ |
| F | Cl | $CH_2CH_3$ | $CH(CH_3)CH_2OCH_3$ |
| H | Cl | H | $CH_2CH=CH_2$ |
| F | Cl | H | $CH_2CH=CH_2$ |
| H | Cl | Cl | $CH_2CH=CH_2$ |
| F | Cl | Cl | $CH_2CH=CH_2$ |
| H | Cl | Br | $CH_2CH=CH_2$ |
| F | Cl | Br | $CH_2CH=CH_2$ |
| H | Cl | $CH_3$ | $CH_2CH=CH_2$ |
| F | Cl | $CH_3$ | $CH_2CH=CH_2$ |
| H | Cl | $CH_2CH_3$ | $CH_2CH=CH_2$ |
| F | Cl | $CH_2CH_3$ | $CH_2CH=CH_2$ |
| H | Cl | H | $CH_2CH=CHCH_3$ |
| F | Cl | H | $CH_2CH=CHCH_3$ |
| H | Cl | Cl | $CH_2CH=CHCH_3$ |
| F | Cl | Cl | $CH_2CH=CHCH_3$ |

Tabelle 1 (Fortsetzung)

| $R^1$ | $R^2$ | $R^3$ | $R^4$ |
|---|---|---|---|
| H | Cl | Br | $CH_2CH=CHCH_3$ |
| F | Cl | Br | $CH_2CH=CHCH_3$ |
| H | Cl | $CH_3$ | $CH_2CH=CHCH_3$ |
| F | Cl | $CH_3$ | $CH_2CH=CHCH_3$ |
| H | Cl | $CH_2CH_3$ | $CH_2CH=CHCH_3$ |
| F | Cl | $CH_2CH_3$ | $CH_2CH=CHCH_3$ |
| H | Cl | H | $CH_2C{\equiv}CH$ |
| F | Cl | H | $CH_2C{\equiv}CH$ |
| H | Cl | Cl | $CH_2C{\equiv}CH$ |
| F | Cl | Cl | $CH_2C{\equiv}CH$ |
| H | Cl | Br | $CH_2C{\equiv}CH$ |
| F | Cl | Br | $CH_2C{\equiv}CH$ |
| H | Cl | $CH_3$ | $CH_2C{\equiv}CH$ |
| F | Cl | $CH_3$ | $CH_2C{\equiv}CH$ |
| H | Cl | $CH_2CH_3$ | $CH_2C{\equiv}CH$ |
| F | Cl | $CH_2CH_3$ | $CH_2C{\equiv}CH$ |
| H | Cl | H | $CH_2C{\equiv}CCH_3$ |
| F | Cl | H | $CH_2C{\equiv}CCH_3$ |
| H | Cl | Cl | $CH_2C{\equiv}CCH_3$ |
| F | Cl | Cl | $CH_2C{\equiv}CCH_3$ |
| H | Cl | Br | $CH_2C{\equiv}CCH_3$ |
| F | Cl | Br | $CH_2C{\equiv}CCH_3$ |
| H | Cl | $CH_3$ | $CH_2C{\equiv}CCH_3$ |
| F | Cl | $CH_3$ | $CH_2C{\equiv}CCH_3$ |
| H | Cl | $CH_2CH_3$ | $CH_2C{\equiv}CCH_3$ |
| F | Cl | $CH_2CH_3$ | $CH_2C{\equiv}CCH_3$ |
| H | Cl | H | $CH_2Ph$ |
| F | Cl | H | $CH_2Ph$ |
| H | Cl | Cl | $CH_2Ph$ |
| F | Cl | Cl | $CH_2Ph$ |
| H | Cl | Br | $CH_2Ph$ |
| F | Cl | Br | $CH_2Ph$ |
| H | Cl | $CH_3$ | $CH_2Ph$ |
| F | Cl | $CH_3$ | $CH_2Ph$ |
| H | Cl | $CH_2CH_3$ | $CH_2Ph$ |
| F | Cl | $CH_2CH_3$ | $CH_2Ph$ |
| H | F | Cl | $CH_3$ |
| H | F | Br | $CH_3$ |
| H | F | $CH_3$ | $CH_3$ |

Tabelle 1 (Fortsetzung)

| $R^1$ | $R^2$ | $R^3$ | $R^4$ |
|---|---|---|---|
| H | F | Cl | $CH_2CH_3$ |
| H | F | Br | $CH_2CH_3$ |
| H | F | $CH_3$ | $CH_2CH_3$ |
| H | F | Cl | $(CH_2)_2CH_3$ |
| H | F | Br | $(CH_2)_2CH_3$ |
| H | F | $CH_3$ | $(CH_2)_2CH_3$ |
| H | F | Cl | $CH(CH_3)_2$ |
| H | F | Br | $CH(CH_3)_2$ |
| H | F | $CH_3$ | $CH(CH_3)_2$ |
| H | F | Cl | $(CH_2)_3CH_3$ |
| H | F | Br | $(CH_2)_3CH_3$ |
| H | F | $CH_3$ | $(CH_2)_3CH_3$ |
| H | F | Cl | $CH_2CH(CH_3)_2$ |
| H | F | Br | $CH_2CH(CH_3)_2$ |
| H | F | $CH_3$ | $CH_2CH(CH_3)_2$ |
| H | F | Cl | $(CH_2)_4CH_3$ |
| H | F | Br | $(CH_2)_4CH_3$ |
| H | F | $CH_3$ | $(CH_2)_4CH_3$ |
| H | F | Cl | $(CH_2)_2CH(CH_3)_2$ |
| H | F | Br | $(CH_2)_2CH(CH_3)_2$ |
| H | F | $CH_3$ | $(CH_2)_2CH(CH_3)_2$ |
| H | F | Cl | $(CH_2)_2OCH_3$ |
| H | F | Br | $(CH_2)_2OCH_3$ |
| H | F | $CH_3$ | $(CH_2)_2OCH_3$ |
| H | F | Cl | $CH(CH_3)CH_2OCH_3$ |
| H | F | Br | $CH(CH_3)CH_2OCH_3$ |
| H | F | $CH_3$ | $CH(CH_3)CH_2OCH_3$ |
| H | F | Cl | $CH_2CH=CH_2$ |
| H | F | Br | $CH_2CH=CH_2$ |
| H | F | $CH_3$ | $CH_2CH=CH_2$ |
| H | F | Cl | $CH_2CH=CHCH_3$ |
| H | F | Br | $CH_2CH=CHCH_3$ |
| H | F | $CH_3$ | $CH_2CH=CHCH_3$ |
| H | F | Cl | $CH_2CH\equiv CH$ |
| H | F | Br | $CH_2CH\equiv CH$ |
| H | F | $CH_3$ | $CH_2CH\equiv CH$ |
| H | F | Cl | $CH_2C\equiv CCH_3$ |
| H | F | Br | $CH_2C\equiv CCH_3$ |
| H | F | $CH_3$ | $CH_2C\equiv CCH_3$ |

Tabelle 1 (Fortsetzung)

| $R^1$ | $R^2$ | $R^3$ | $R^4$ |
|---|---|---|---|
| H | F | Cl | $CH_2Ph$ |
| H | F | Br | $CH_2Ph$ |
| H | F | $CH_3$ | $CH_2Ph$ |

Die N-Phenyltetrahydroindazolderivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.001 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.001 werden in einer Mischung gelöst, die aus 40 Gewichtsteilen

Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.001 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.002 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.002 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.002 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 0,5 kg/ha.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |

| Botanischer Name | Deutscher Name |
|---|---|
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |

| Botanischer Name | Deutscher Name |
|---|---|
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Phenyltetrahydroindazolderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der nachstehenden Tabelle 2 mit physikalischen Daten aufgeführt.

Beispiel 1

Herstellung von E/Z-3-Chlor-2-[3-(2-methoxycarbonylprop-1-enyl)-4-chlorphenyl ]-4,5,6,7 -tetrahydroindazol

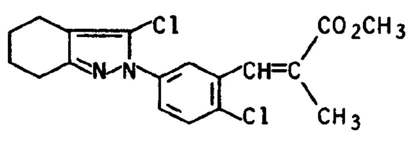

a) Eine Mischung aus 15,4 g (72 mmol) 2-Chlor-5-amin-α-methylzimtsäure, 35 ml Essigsäure und 21 ml konzentrierter Salzsäure wurde bei 0 bis 5°C langsam mit einer Lösung aus 5 g (72 mmol) Natriumnitrit und 14 ml Wasser versetzt. Nach 30 Minuten Rühren bei 5°C wurden zuerst 150 ml konzentrierte Salzsäure und anschließend 32,4 g (143 mmol) $SnCl_2$ x $2H_2O$ in 30 ml konz. HCl zugetropft. Das erhaltene Reaktionsgemisch wurde anschließend 12 Stunden bei Raumtemperatur gerührt und der gebildete Niederschlag

isoliert. Man erhielt E/Z-3-(2-Hydroxycarbonyl-prop-1-enyl)-4-chlorphenylhydrazin in quantitativer Ausbeute (Öl).

b) 11,3 g (50 mmol) des Phenylhydrazins aus a) wurden zu einer Lösung aus 9,35 g (55 mmol) Cyclohexanon-2-carbonsäureethylester, 100 ml Eisessig, 4,1 g (50 mmol) Natriumacetat und 10 ml Wasser gegeben. Nach 6 Stunden bei Siedetemperatur und Abkühlen auf 25°C wurde der gebildete Feststoff isoliert. Man erhielt 5,8 g (35 %) E/Z-2-[3(-2-Hydroxycarbonylprop-1-enyl)-4-chlorphenyl]-1,2,4,5,6,7-hexahydro-3H-indazol-3-on (Fp. 278°C).

c) Eine Mischung aus 5,6 g (17 mmol) des Indazolons aus b) und 850 mg Dimethylformamid wurde bei 25°C mit 12,9 g (84 mmol) Phosphoroxichlorid versetzt. Nach 30 Minuten bei 25°C wurde für 1 Stunde bei Siedetemperatur gerührt. Danach wurde das überschüssige Phosphoroxichlorid abdestilliert und der Rückstand bei 25°C unter Kühlung mit 54 ml Methanol und nach 15 Minuten mit 5,3 g (67 mmol) Pyridin versetzt. Nach 12 Stunden Rühren bei 25°C wurde die Mischung eingeengt, der Rückstand in 100 ml Essigester aufgenommen, gewaschen und getrocknet. Man erhielt nach chromatographischer Reinigung 3,3 g (53 %) der Titelverbindung als Öl (Wirkstoffbeispiel 1.001).

Tabelle 2

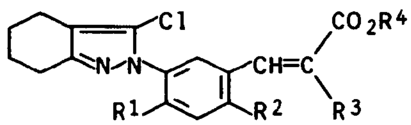

I

| Wirkstoff Nr. | $R^1$ | $R^2$ | $R^3$ | $R^4$ | physik. Daten [Fp (°C); IR ($cm^{-1}$); NMR (ppm)] |
|---|---|---|---|---|---|
| 1.001 | H | Cl | $CH_3$ | $CH_3$ | NMR: 2,05(s); 3,85(s) |
| 1.002 | H | Cl | $CH_3$ | $CH_2CH_3$ | NMR: 2,05(s); 4,35(q) |
| 1.003 | H | Cl | Br | $CH_3$ | Fp.: 83-85 |
| 1.004 | H | Cl | Br | $CH_2CH_3$ | Fp.: 70-71 |
| 1.005 | H | Cl | Cl | $CH_2CH_3$ | Fp.: 57-58 |
| 1.006 | H | Cl | Cl | $CH_3$ | Fp.: 71-72 |
| 1.007 | H | Cl | Br | $C(CH_3)_3$ | IR : 2936,1723,1156,1006 |
| 1.008 | H | Cl | Cl | $C(CH_3)_3$ | IR : 2936,1726,1158,1016 |
| 1.009 | H | Cl | Cl | $CH(CH_3)_2$ | Fp.: 64-65 |
| 1.010 | H | Cl | Br | $CH(CH_3)_2$ | Fp.: 84-85 |
| 1.011 | H | Cl | I | $CH_3$ | Fp.: 91-92 |

Anwendungsbeispiele

Die Wirkung der N-(Phenyl)tetrahydroindazolderivate I auf das Wachstum der Testpflanzen ließ sich durch folgende Gewächshausversuche zeigen.

Zur Anzucht der Testpflanzen dienten Plastikblumentöpfe mit 300 $cm^3$ Inhalt mit lehmigem Sand und etwa 3,0 % Humus als Substrat. Die Samen der Testpflanzen wurden nach Arten getrennt eingesät.

Zum Zweck der Nachauflaufbehandlung wurden entweder direkt gesäte oder in gleichen Gefäßen aufgewachsene Pflanzen ausgewählt, oder sie wurden erst als Keimpflanzen getrennt angezogen und einige Tage vor der Behandlung in die Versuchsgefäße verpflanzt.

Je nach Wuchsform wurden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt wurden, behandelt. Die Aufwandmenge für die Nachauflaufbehandlung betrug 0,06 kg/ha a.S. (aktive Substanz).

Die Versuchsgefäße wurden im Gewächshaus aufgestellt, wobei für wärmeliebende Arten wärmere Bereiche (20 bis 35°C) und für solche gemäßigter Klimate 10 bis 20°C bevorzugt wurden. Die Versuchsperiode erstreckte sich über 2 bis 4 Wochen. Während dieser Zeit wurden die Pflanzen gepflegt und ihre Reaktion auf

die einzelnen Behandlungen ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Chenopodium albun | Weißer Gänsefuß |
| Solanum nigrum | Schwarzer Nachtschatten |
| Triticum aestivum | Sommerweizen |

Bei Aufwandmengen von 0,06 kg/ha a.S. ließen sich im Nachauflaufverfahren eingesetzt mit den Verbindungen 1.001 und 1.002 breitblättrige unerwünschte Pflanzen sehr gut bekämpfen, während die Beispielkultur Triticum aestivum nicht nennenswert geschädigt wurde.

**Patentansprüche**

1. N-Phenyltetrahydroindazolderivate der allgemeinen Formel I

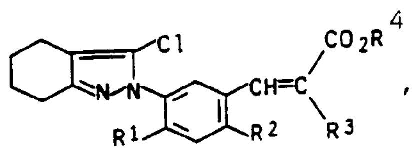

Ib

in der die Substituenten folgende Bedeutung haben:

$R^1$ Wasserstoff oder Fluor,

$R^2$ ein Halogenatom,

$R^3$ Wasserstoff, ein Halogenatom oder eine $C_1$-$C_4$-Alkylgruppe,

$R^4$ Wasserstoff, eine $C_1$-$C_6$-Alkylgruppe, welche durch ein oder zwei $C_1$-$C_4$-Alkoxygruppen substituiert sein kann, eine $C_3$-$C_6$-Alkenylgruppe, eine $C_3$-$C_6$-Alkinylgruppe oder ein Benzylrest.

2. Verfahren zur Herstellung der Verbindungen I gemäß Anspruch 1, dadurch gekennzeichnet, daß man ein Indazolzimtsäurechlorid VII

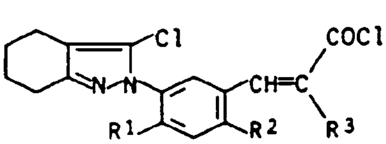

VII

in an sich bekannter Weise in einem aprotisch polaren inerten organischen Lösungsmittel in Anwesenheit einer Base mit einer Verbindung VIII

$HOR^4$ VIII

zum N-Phenyltetrahydroindazolderivat I hydrolysiert bzw. verestert.

3. Verfahren zur Herstellung der Verbindungen I gemäß Anspruch 1, dadurch gekennzeichnet, daß man ein entsprechendes Nitrozimtsäurederivat II

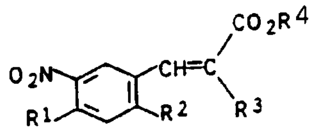

II

in an sich bekannter Weise in einem inerten organischen Lösungsmittel bei Temperaturen bis 200°C zum entsprechenden Anilinderivat III

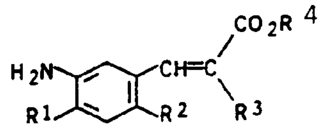

III

reduziert, dieses nach allgemein üblichen Methoden mit einem anorganischen oder organischen Nitrit diazotiert und in an sich bekannter Weise in situ mit einem anorganischen Reduktionsmittel zum Hydrazinzimtsäurederivat IV

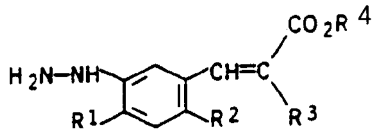

IV

umsetzt, dieses daraufhin nach bekannten Methoden in einem inerten organischen Lösungsmittel bei Temperaturen bis 200°C mit einem Cyclohexanoncarbonsäurederivat V

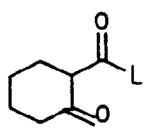

V

wobei L eine nucleophile Abgangsgruppe bedeutet, zum Imidazolzimtsäurederivat VI

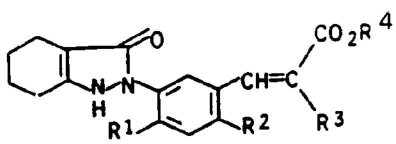

VI

cyclisiert und anschließend mit einem Chlorierungsmittel zum N-Phenyltetrahydroindazolderivat I chloriert.

4. Verwendung der N-Phenyltetrahydroindazolderivate der Formel I gemäß Anspruch 1 als Herbizide.

5. Herbizide Mittel, enthaltend ein N-Phenyltetrahydroindazolderivat der Formel I gemäß Anspruch 1 und inerte Zusatzstoffe.

6. Herbizide Mittel nach Anspruch 5, enthaltend ein N-Phenyltetrahydroindazolderivat der Formel I gemäß Anspruch 1 und weitere wirksame Bestandteile.

7. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines N-Phenyltetrahydroindazolderivates I gemäß Anspruch 1 behandelt.

## Claims

1. A N-phenyltetrahydroindazole derivative of the formula I

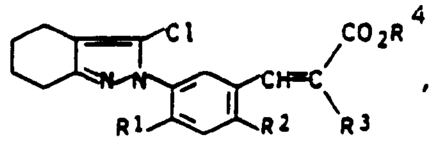,

Ib

where

$R^1$ is hydrogen or fluorine,
$R^2$ is halogen,
$R^3$ is hydrogen, halogen or $C_1$-$C_4$-alkyl,
$R^4$ is hydrogen, $C_1$-$C_6$-alkyl which can be substituted by one or two $C_1$-$C_4$-alkoxy groups, or is $C_3$-$C_6$-alkenyl, $C_3$-$C_6$-alkynyl or benzyl.

2. A process for preparing a compound I as claimed in claim 1, wherein a tetrahydroindazolecinnamyl chloride VII

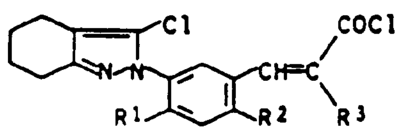

VII

is hydrolyzed or esterified in conventional manner in an aprotic polar inert organic solvent in the presence of a base with a compound VIII

$HOR^4$ VIII

to give an N-phenyltetrahydroindazole derivative I.

3. A process for preparing a compound I as claimed in claim 1, wherein a corresponding nitrocinnamic acid derivative II

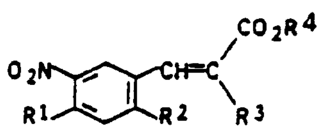

II

is reduced in conventional manner in an inert organic solvent at a temperature of up to 200° C to give the corresponding aniline derivative III

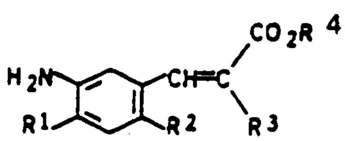

III,

which is diazotized by generally conventional methods with an inorganic or organic nitrite and is reacted in conventional manner in situ with an inorganic reducing agent to give the hydrazinocinnamic acid derivative IV

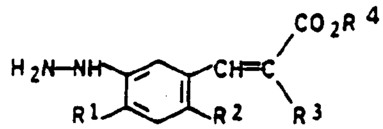

IV,

which is then cyclized by known methods in an inert organic solvent at a temperature of up to 200°C with a cyclohexanonecarboxylic acid derivative V

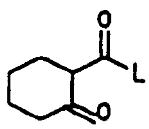

V,

where L is a nucleophilic leaving group, to give the imidazolecinnamic acid derivative VI

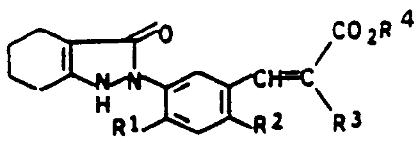

VI,

and then chlorinated with a chlorinating agent to give the N-phenyltetrahydroindazole derivative I.

4. The use of an N-phenyltetrahydroindazole derivative of the formula I as claimed in claim 1 as a herbicide.

5. A herbicidal agent containing an N-phenyltetrahydroindazole derivative of the formula I as claimed in claim 1, and inert additives.

6. A herbicidal agent as claimed in claim 5, containing an N-phenyltetrahydroindazole derivative of the formula I as claimed in claim 1 and further active constituents.

7. A process for controlling the growth of undesirable plants, wherein the undesirable plants and/or their habitat are treated with a herbicidally effective amount of an N-phenyltetrahydroindazole derivative I as claimed in claim 1.

**Revendications**

1. Dérivés du N-phényltétrahydro-indazole de formule générale I

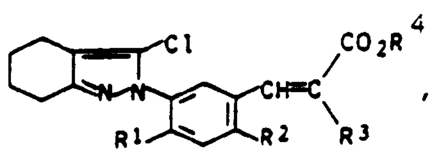

,

Ib

dans laquelle les symboles ont les significations suivantes :

$R^1$ représente l'hydrogène ou le fluor,

$R^2$ représente un atome d'halogène,

$R^3$ représente l'hydrogène, un atome d'halogène ou un groupe alkyle en C1-C4,

$R^4$ représente l'hydrogène, un groupe alkyle en C1-C6 qui peut être substitué par un ou deux groupes alcoxy en C1-C4, un groupe alcényle en C3-C6, un groupe alcynyle en C3-C6 ou un groupe benzyle.

2. Procédé de préparation des composés I de la revendication 1, caractérisé en ce que l'on hydrolyse ou on

estérifie respectivement un chlorure d'acide tétrahydro-indazole-cinnamique VII

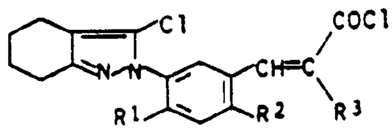 VII

de manière connue en soi, dans un solvant organique polaire aprotonique inerte, en présence d'une base, à l'aide d'un composé VIII

$HOR^4$ VIII

la réaction donnant le dérivé de N-phényltétrahydroindazole I.

3. Procédé de préparation des composés I de la revendication 1, caractérisé en ce que l'on réduit un dérivé correspondant d'acide nitrocinnamique II

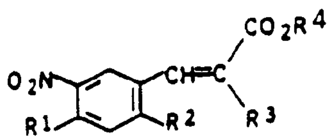 II

de manière connue en soi, dans un solvant organique inerte, à des températures allant jusqu'à 200 degrés C, en le dérivé correspondant d'aniline III

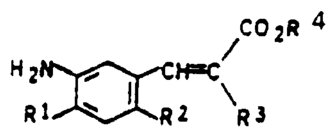 III

qu'on diazote par des modes opératoires généraux usuels, à l'aide d'un nitrite minéral ou organique, on fait réagir de manière connue en soi, in situ, avec un agent réducteur minéral, ce qui donne le dérivé d'acide hydrazine-cinnamique IV

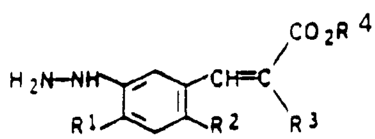 IV

lequel est cyclisé par des modes opératoires connus, dans un solvant organique inerte, à des températures allant jusqu'à 200 degrés C, à l'aide d'un dérivé d'acide cyclohexanone-carboxylique de formule V

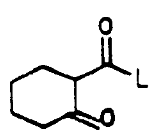 V

dans laquelle L représente un groupe éliminable nucléophile, ce qui donne le dérivé imidazole-cinnamique VI

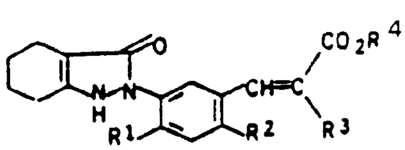 VI

qu'on chlore à l'aide d'un agent chlorant, cette réaction donnant le dérivé de N-phényltétrahydro-indazole I.

4. Utilisation des dérivés de N-phényltétrahydro-indazole de formule I de la revendication 1 en tant qu'herbicides.

5. Produits herbicides contenant un dérivé de N-phényltétrahydro-indazole de formule I de la revendication 1 et des additifs inertes.

6. Produits herbicides selon la revendication 5, contenant un dérivé de N-phényltétrahydro-indazole de formule I de la revendication 1 et d'autres composants actifs.

7. Procédé pour combattre les croissances de végétaux indésirables, caractérisé en ce que l'on traite les végétaux indésirables et/ou leur habitat par une quantité herbicide efficace d'un dérivé de N-phényltétrahydro-indazole I de la revendication 1.